# EUROPEAN PATENT SPECIFICATION

(11) **EP 1 226 153 B1**
(45) Date of publication and mention of the grant of the patent: **03.03.2010**
(21) Application number: 00973834.5
(22) Date of filing: 26.10.2000
(51) Int. Cl.: C07H 23/00, A61K 49/00, G01N 33/53

(54) **FLUORESCENT COBALAMINS AND USES THEREOF**
FLUORESZIERENDE COBALAMINE UND DEREN VERWENDUNG
COBALAMINES FLUORESCENTES ET UTILISATIONS

(30) Priority: 26.10.1999 US 161368 P
(43) Date of publication of application: 31.07.2002
(73) Proprietor: University of Utah Research Foundation, Salt Lake City, Utah 84108 (US)
(72) Inventor: GRISSOM, Charles, B., Salt Lake City, UT 84108 (US); WEST, Frederick, G., Salt Lake City, UT 84105 (US); MCGREEVY, James, Salt Lake City, UT 84121-6308 (US); BENTZ, Joel, S., Salt Lake City, UT 84121 (US)
(74) Representative: Rambelli, Paolo
(86) International application number: PCT/US2000/029370
(87) International publication number: WO 2001/030967

(56) References cited:
- EP-A- 0 069 450
- US-A- 5 614 394
- US-A- 5 739 287
- US-A- 5 840 712
- COLLINS ET AL.: 'Transcobalamin II receptor imaging via radiolabeled diethylene-triaminepentaacetate cobalamin analogs' THE JOURNAL OF NUCLEAR MEDICINE vol. 38, no. 5, May 1997, pages 717 - 723, XP002940342
- CARMEL: 'Extreme elevation of serum transcobalamin I in patients with metastatic cancer' THE NEW ENGLAND JOURNAL OF MEDICINE vol. 292, no. 6, 06 February 1975, pages 282 - 284, XP002940341

## Description

### BACKGROUND OF THE INVENTION

The present invention relates to cobalamins and uses of these compounds. More particularly, this invention relates to cobalamins comprised of a fluorescent, phosphorescent, luminescent or light-producing compound that is covalently linked to cobalamin. In the following description, such cobalamins are all together named as fluorescent cobalamins. These fluorescent cobalamins can be used as diagnostic and prognostic markers (a) to distinguish cancer cells and tissues from healthy cells and tissues and (b) to determine if an individual will respond positively to chemotherapy using cobalamin-based therapeutic bioconjugates.

The publications and other materials used herein to illuminate the background of the invention, and in particular cases, to provide additional details respecting the practice, are incorporated by reference, and for convenience are referenced in the following text by author and date and are listed alphabetically by author in the appended bibliography.

Rapidly-dividing cells require cobalamin as a cofactor for the enzyme methionine synthase to support one-carbon metabolism prior to DNA replication (Hogenkamp et al., 1999). In acute promyelocytic leukemia, a 3-26 fold increase in the unsaturated B₁₂ binding capacity of blood is observed, due to an increase in the concentration of the B₁₂ binding proteins transcobalamin and haptocorrin (Schneider, et al., 1987; Rachimelwitz, et al., 1971). Some patients with solid tumors also exhibit a significant increase in the circulating levels of transcobalamin and haptocorrin (Carmel, et al., 1975). The increase in unsaturated serum cobalamin binding capacity corresponds to the increased uptake of cobalamin by rapidly dividing cells. Tumors even sequester sufficient cobalamin for diagnostic imaging purposes if a gamma-emitting radionuclide, such as ¹¹¹In, is attached to cobalamin through the octadentate chelator diethylenetriaminepentaacetic acid (DTPA) (Hogenkamp and Collins, 1997). This has been demonstrated in mice with an implanted fibrosarcoma (Hogenkamp and Collins, 1997), as well as in humans with breast cancer (Collinset al., 1999), and in tumors of the prostate, lung, and brain (Collins et al., 2000).

In the sentinel lymph node concept for melanoma and breast cancer surgery, a dye or radionuclide is injected into the tissue around the tumor to identify the first lymph node that drains the tumor (Morton et al., 1992; McGreevy, 1998). This node is termed the sentinel node, and it is removed for diagnostic tests to determine the extent of metastasis beyond the primary tumor. This procedure is controversial, as it fails to detect metastatic disease in about 12% of patients (McMasters et al., 1999). The dye or radionuclide that is injected is not specific for cancer cells, but merely identifies for the surgeon the primary lymph node that drains the region of the tumor. The high false-negative rate should be improved dramatically by using a fluorescent marker that is specific for cancer cells.

Thus, there exists a need for an agent that can be used for the diagnosis and prognosis of cancer tissue or cells with improved results.

### SUMMARY OF THE INVENTION

The subject matter of the present invention is cobalamins and uses of these compounds, as defined in the appended claims. More particularly, this invention relates to fluorescent Cobalamins comprised of a fluorescent, phosphorescent luminescent or light-producing compound that is covalently linked to cobalamin, according to the general formula (I) shown hereinafter. These fluorescent cobalamins can be used as a diagnostic and prognostic marker (a) to distinguish cancer cells and tissues from healthy cells and tissues and (b) to determine if an individual will respond positively to chemotherapy using cobalamin-therapeutic bioconjugates. The fluorescent cobalamins of the present invention offer the necessary properties of (1) rapid transport and storage by cancer cells (maximum uptake occurs at 4-6 hours), (2) a bright fluorophore that can be visually detected at very low concentrations, and (3) nontoxic components.

In one aspect of the present invention, fluorescent cobalamins are provided in which fluorescent, phosphorescent, luminescent or light-producing compounds are covalently linked to cobalamin (vitamin B₁₂), as shown in formulas (I). The fluorescent, phosphorescent or light-producing compounds can be covalently linked to the cobalt atom, the corrin ring, or the ribose moiety of cobalamin. It is preferred to covalently link the fluorescent, phosphorescent, luminescent or light-producing compound to the corrin ring or the ribose moiety. Although, any fluorescent, phosphorescent, luminescent or light-producing compound can be utilized in preparing the fluorescent cobalamins, it is preferred to utilize fluorescent, phosphorescent, luminescent or light-producing compounds that are excitable with visible or infrared light. Examples of preferred fluorescent compounds include, but are not limited to, fluorescein, fluorescein-5EX, methoxycoumarin, naphthofluorescein, BODIPY 493/503, BODIPY FL, BODIPY R6G, BODIPY 530/550, BODIPY TMR, BODIPY 564/570, BODIPY 576/589, BODIPY 581/591, BODIPY TR, Cascade Blue, Dansyl, Dialkylaminocoumarin, 4',5'-dichloro-2',7'-dimethyoxyfluorescein, 2',7'-dichlorofluorescein, eosin, eosin F3S, erythrosin, hydroxycoumarin, lissamine rhodamine B, methosycoumarin, maphthofluorescein, NBD, Oregon Green 488, Oregon Green 500, Oregon Green 514, PyMPO, pyrene, rhodamine 6G, rhodamine green, rhodamin red, rhodol green, 2',4',5',7'-tetrabromosulfonefluorescein, tetramethylrhodamine (TMR), Texas Red, X-rhodamine, Cy2 dye, Cy3 dye, Cy5 dye, Cy5.5 dye, or a quantum dot structure. The preferred fluorescent cobalamins of the present invention fluoresce when excited by visible or infrared light without the need to separate the fluorescent or phosphorescent compound from cobalamin.

In a second aspect of the present invention, the fluorescent cobalamins are used to distinguish cancer cells from healthy cells. In one embodiment of this aspect of the invention, a fluorescent cobalamin is administered to a patient prior to surgery. The presence of fluorescence, phosphorescence, luminescence or emited light in cancer cells is used by the surgeon to define the tissue to be removed, whether in a primary tumor or in a metastatic site. In a second embodiment, a fluorescent cobalamin is administered to a patient in a manner suitable for uptake by lymph nodes draining the *situs* of the tumor. The presence of fluorescence, phosphorescence, luminescence or emited light identifies those lymph nodes that should be removed during surgery.

In a third aspect of the present invention, the fluorescent cobalamins are used to determine if an individual will respond positively to chemotherapy using cobalamin-based therapeutic bioconjugates. In this aspect, a fluorescent cobalamin is used to assess the ability of the particular cancer cell type to transport and store cobalamin, both qualitatively and quantitatively. Various types of cancer that transport and store large amounts of cobalamin are good candidates for therapy with cobalamin-based therapeutic bioconjugates. Quantification of tumor cell cobalamin binding, uptake, transport, and storage can be carried out by measuring the fluorescence under visual inspection (e.g. tissue slide), by epifluorescence microscopy, or by flow cytometry.

In a fourth aspect of the present invention, the fluorescent cobalamins are used to determine the levels of cobalamin in blood, plasma, serum, cerebrospinal fluid or urine or to determine the amount of unbound cobalamin binding capacity iin blood, plasma, serum or cerebrospinal fluid.

### BRIEF DESCRIPTION OF THE DRAWINGS

Figure 1 shows the synthesis of one fluorescent cobalamin in accordance with the present invention.
Figure 2 shows the synthesis of cobalamin monocarboxylic acids.
Figure 3 shows the conjugation of cobalamin carboxylic acids with 1,12-diaminododecane.

### DETAILED DESCRIPTION OF THE INVENTION

The present invention relates to fluorescent cobalamins and uses of these compounds. More particularly, this invention relates to fluorescent cobalamins that comprise a fluorescent compound (fluorophore), a phosphorescent compound (phosphorophore), a luminescent compound (chemiluminescent chromophore) or a light-producing compound that is covalently linked to cobalamin (vitamin B₁₂). These fluorescent cobalamins can be used as diagnostic and prognostic markers (a) to distinguish cancer cells and cancerous tissue from healthy cells and tissues and (b) to determine if an individual will respond positively to chemotherapy using cobalamin-therapeutic bioconjugates.

The fluorescent cobalamins of the present invention can be represented by the following formula 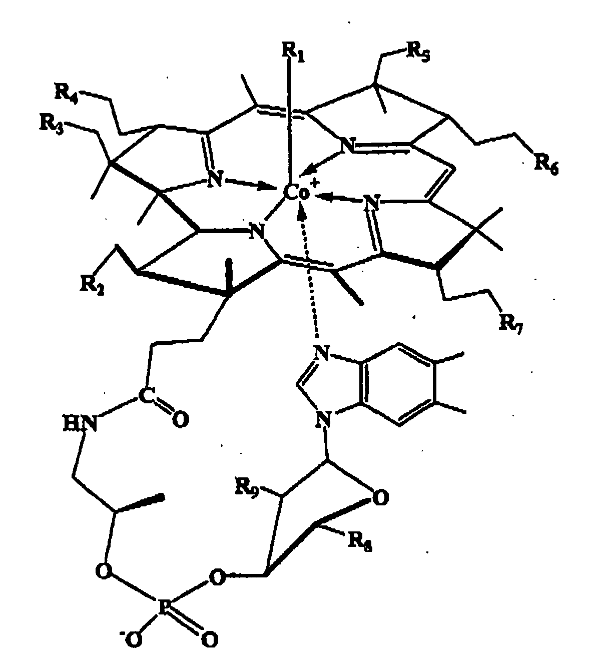 where R₁ is CN, OH, OH₂. CH₃, 5'-deoxyadenosine or (CH₂)ₚNHC(=S)Y; R₂, R₃, R₄, R₅, R₆, and R₇ are CONCH₂ R₈ is CH₂OH or O(C=O)XₘY; R₉ is OH or O(C=O)XₘY; X is a linker having the formula NH(CH₂)ₙNHO(C=O); Y is a fluorophore, a phosphorophore, or a chemiluminescent chromophore; m is 0 or 1, n is 0-50 and p is 2-10, with the proviso that at least one of R₁, R₈ and R₉ groups has Y, wherein said cobalamin fluoresces, phosphoresces, or luminesces when illuminated with ultraviolet, visible or infrared light without cleavage of Y from the cobalamin.

The fluorescent cobalamins of the present invention are prepared by covalently attaching a fluorophore, a phosphorophore or a chemiluminescent chromophore to cobalamin. The fluorophore, phosphorophore or chemiluminescent chromophore is covalently linked to the cobalt atom, or to the ribose sugar directly or via a linker molecule as defined above. The covalent linkage is preferably accomplished with the use of said linker molecule. If the fluorophore, phosphorophore or chemiluminescent chromophore is attached to the cobalt atom of cobalamin, the fluorescence, phosphorescence or emitted light is diminished in intensity through quenching by the spin of the cobalt atom. In addition, prolonged exposure of the fluorescent cobalamin to light will cleave the cobalt-carbon bond and release the fluorophore, phosphorophore or chemiluminescent chromophore or from cobalamin (Howard et al., 1997). Thus, it is preferred to attach the fluorophore, phosphorophore or chemiluminescent chromophore to the ribose moiety of the cobalamin molecule. These latter fluorescent cobalamins do not have the disadvantages of the fluorescent cobalamins in which the fluorophore, phosphorophore or chemiluminescent chromophore is covalently linked to the cobalt atom.

Although, any fluorophore, phosphorophore or chemiluminescent chromophore can be utilized in preparing the fluorescent cobalamins, it is preferred to utilize fluorophores that are excitable with visible or infrared light. It is preferred to use visible or infrared light for *in vivo* use of the fluorescent cobalamins. Examples of preferred fluorophores include, but are not limited to, fluorescein, fluorescein-5EX, methoxycoumarin, naphthofluorescein, BODIPY 493/503, BODIPY FL, BODIPY R6G, BODIPY 530/550, BODIPY TMR, BODIPY 564/570, BODIPY 576/589, BODIPY 581/591, BODIPY TR, Cascade Blue, Dansyl, Dialkylaminocoumarin, 4',5'-dichloro-2',7'-dimethyoxyfluorescein, 2',7'-dichlorofluorescein, eosin, eosin F3S, erythrosin, hydroxycoumarin, lissamine rhodamine B, methosycoumarin, maphthofluorescein, NBD, Oregon Green 488, Oregon Green 500, Oregon Green 514, PyMPO, pyrene, rhodamine 6G, rhodamine green, rhodamin red, rhodol green, 2',4',5',7'-tetrbromosulfonefluorescein, tetramethylrhodamine (TMR), Texas Red, X-rhodamine, Cy2 dye, Cy3 dye, Cy5 dye, Cy5.5 dye, or a quantum dot structure. The preferred fluorescent cobalamins of the present invention fluoresce when excited by visible or infrared light without the need to cleave the fluorophore from the bioconjugate.

It has been found that there is differential uptake of fluorescent cobalamin analogues in normal and leukemic human bone marrow. The difference between normal marrow cells and leukemic myeloblasts (cancer cell) is particularly noteworthy, with no detectable cobalamin being taken up by normal cells. Bone marrow samples from healthy individuals show no fluorescent labeling. It has also been found that there is uptake of a doxorubicin-cobalamin conjugate, originally synthesized as a potential chemotherapeutic compound. Cellular uptake of the doxorobicin-cobalamin conjugate can be observed in P-388 murine leukemia cells, as well as in HCT-116 human colon tumor cells. Thus, the uptake of fluorescent derivatives of cobalamin occurs in leukemia and solid tumor cell lines. These results, in combination with the knowledge that all cancer cells increase cobalamin transport and storage, demonstrate the general applicability of the use of fluorescent cobalamins to distinguish cancer cells from normal cells.

Thus, the fluorescent cobalamins of the present invention can be used to:
- identify cancerous tissue visually, via fluorescence microscopy, or by flow cytometry;
- identify cancerous cells in tissue sections or samples from tissue biopsies;
- define tumor margins *in vivo*, *ex vivo* or *in situ*;
- diagnose, detect, prognose, predict or monitor cancer *in vivo*, *ex vivo* or *in situ*;
- identify metastatic cancer *in vivo*, *ex vivo* or *in situ*;
- determine the stage of cancer progression;
- identify cancer transdermally;
- identify metastatic cancer transdermally;
- identify cancer in lymph nodes, including in the sentinel lymph node or nodes or in an axillary lymph node or nodes;
- identify metastatic disease in the treatment, detection, prediction, prognostication or monitoring of cancer, such as breast cancer, ovarian cancer, lung cancer, prostate cancer, epithelial cancer (adenocarcinoma), liver cancer, melanoma and lymphoma;
- conduct flow cytometry studies of bone marrow aspirates or peripheral blood samples for diagnosing, predicting, prognosticating, monitoring or characterizing leukemia or lymphoma;
- predict whether a patient will respond positively to chemotherapy that is based on the use of a cobalamin-therapeutic bioconjugate;
- improve the definition of tumor micromargins in a biopsy or lumpectomy;
- decrease the chance of leaving cancerous cells behind in a biopsy, lumpectomy, or tumorectomy and thereby reduce the need for follow-up surgery to remove the remaining cancer cells.

Prediction refers to understanding the biological behavior of the tumor, and how the tumor will respond (favorably or unfavorably) to therapy. Prognosis refers to the anticipated patient outcome following therapy (i.e. what is the likelihood of five- or ten-year survival following therapy). Monitoring refers to determining the success of therapy and detection of residual disease following treatment. An example is the use of a fluorescent cobalamin conjugate to test the bone marrow for the presence of myeloblasts following treatment of leukemia. Characterization refers to a descriptive or quantitative classification of the type of tumor in comparison to closely related types of tumors.

The fluorescent cobalamins of the present invention can be administered in accordance with customary cancer diagnostic, detection, prediction, prognostication, monitoring or characterization methods known in the art. For example, the fluorescent cobalamins can be administered intravenously, intrathecally, intratumorally, intramuscularly, intralymphatically, or orally. Typically, an amount of the fluorescent cobalamin of the present invention will be admixed with a pharmaceutically acceptable carrier. The carrier may take a wide variety of forms depending on the form of preparation desired for administration, e.g., oral, parenteral, intravenous, intrathecal, intratumoral, circumtumoral, and epidural. The compositions may further contain antioxidizing agents, stabilizing agents, preservatives and the like. Examples of techniques and protocols can be found in *Remington's Pharmaceutical Sciences*. The amount of fluorescent cobalamin to be administered will typically be 1-500 mg.

An improvement in the surgeon's ability to identify metastatic disease in lymph nodes will advance surgical therapy by preserving, e.g., healthy tissue and minimizing the number of axillary lymph nodes removed. This will improve the patient's quality of life and improve morbidity and long-term mortality. Precise identification of cancer cells that have spread to lymph nodes will allow removal of only the diseased ducts and nodes, while sparing the healthy axillary nodes. This invention is extremely valuable. For example, with 186,000 new cases of breast cancer each year, the number of surgeries to remove primary tumors and determine the status of associated lymph nodes is significant. The perfunctory removal of all axillary lymph nodes and ducts leads to local edema and increased morbidity. The non-removal of axillary lymph nodes and ducts that contain metastatic cancer cells leads to decreased survival and increased long-term mortality.

In the sentinel lymph node biopsy approach, a blue dye and/or radioactive tracer are injected into the breast near the tumor, A small incision is made under the arm to look for traces of the dye or radioactivity to identify the lymph node(s) that drain the area of the breast and, as a consequence, are most likely to contain metastatic cancer cells. In accordance with the present invention, a fluorescent cobalamin replaces the blue dye and radioisotope tracer currently used in sentinel lymph node biopsies. The use of the fluorescent cobalamins of the present invention may enable the future application of the sentinel lymph node biopsy approach to all types of cancer.

In addition, since the fluorescent cobalamins of the present invention are differentially taken up by cancer cells, these fluorescent cobalamins are an improved marker that will allow surgeons to excise cancerous tissue selectively, thereby leaving healthy tissue.

The fluorescent cobalamins of the present invention offer several improvements as an intraoperative marker. These improvements include:
- The fluorescent marker will be specific for cancer cells in lymph ducts and nodes, rather than simply indicating which node is draining the tidal basin. The fluorescent marker will also distinguish cancer cells from healthy cells.
- The marker can be used in low concentrations because of the inherent sensitivity afforded by fluorescence detection. The blue dye now in use tends to obscure the active node and complicates postsurgical examination of the tissue by a pathologist. The blue dye also tends to obscure bleeding vessels, thereby complicating surgical excision of the node and subsequent wound closure. The use of a fluorescent marker should avoid these problems.
- A fluorescent marker that is specific for cancer cells will improve the false-negative rate of 5-10%, as is seen with the procedure as currently practiced.
- A decreased false-negative rate would improve the acceptance of this technique by patients and surgeons. This might decrease the training time necessary (typically 30 or more cases with complete axial node dissection) for a surgeon to learn this procedure.

In a further embodiment of the present invention, the fluorescent cobalamins can be used in a competitive binding assay to determine the concentration or amount of naturally-occurring cobalamin (hydroxocobalamin, methylcobalamin, adenosylcobalamin, or cyanocobalamin) in blood, plasma, serum, or other bodily fluids. In this type of assay, a fluorescent cobalamin is used in place of radioactively-labelled cobalamin in a competitive binding assay, well known to a skilled artisan. Radioactive assays for cobalamin have been described in U.S. Patent Nos. 6,096,290; 5,614,394; 5,227,311; 5,187,107; 5,104,815; 4,680,273; 4,465,775; 4,355.018, among others, each incorporated herein by reference. This assay procedure can be used to determine the amount of unsaturated cobalamin binding capacity in blood, plasma, serum, or bodily fluids, as well as the concentration of cobalamin that is bound to the proteins transcobalamin, haptocorrin, or intrinsic factor. The use of fluorescent cobalamins has a significant advantage over radioacdvely-labelled cobalamin in a clinical chemistry binding assay because it does not require the special shipping, handling, and disposal procedures associated with radioactively-labelled cobalamin.

### EXAMPLES

The present invention is further described by reference to the following Examples, which are offered by way of illustration and are not intended to limit the invention in any manner. Standard techniques well known in the art or the techniques specifically described below were utilized.

### EXAMPLE 1

### Synthesis of Fluorescent Cobalamin by Attachment of the Fluorophore to Cobalt

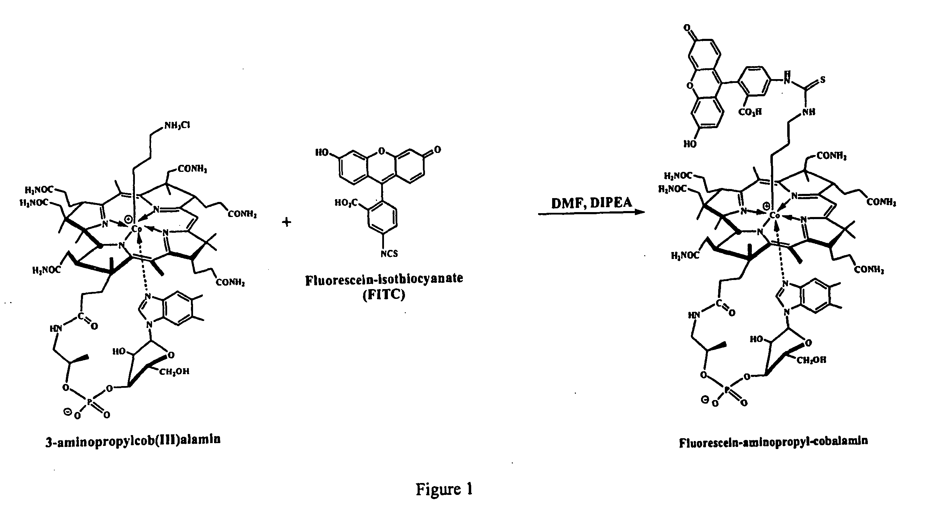

As a visual indicator of cobalamin localization, five fluorescent analogues of cobalamin were prepared by covalently attaching fluorescein to cobalamin. Under green light illumination, the fluorescein molecule emits yellow light that can be detected by the dark-adapted eye to concentrations lower than 0.1 ppm. This emission enables the sensitive detection of cancer cells via epifluorescence microscopy, as well as by visual inspection. Each of the five fluorescent cobalamins exhibited intrinsic fluorescence. All of these compounds were synthesized by reacting aminopropyl chloride with cob(I)alamin to produce aminopropylcob(III)alamin in accordance with published techniques. In a subsequent step, aminopropylcob(in)alamin was reacted with a variety of fluorophore isothiocyanates (i.e. fluorescein isothiocyanate, "FITC") to produce the corresponding fluorophore that is linked to cobalamin through an aminopropyl linker (i.e. fluorescein-aminopropyl-cob(III)alamin) This latter reaction is shown in Figure 1.

In a similar manner, fluorescent cobalamins were prepared in which the fluorophore is naphthofluorescein or Oregon Green. All the fluorescent cobalamins were found to retain high affinity for recombinant transcobalamin (rhTCII), thus allowing for a biological distribution similar to that observed fro naturally occurring cobalamin.

### EXAAELE 2

### Uptake of Cobalamin Analogues by Cancer Cells

A leukemic myeloblast preparation was made from a bone marrow aspirate of a 61-year old patient having acute myelogenous leukemia (AML) M1 (minimally mature myeloblasts in the FLAB classification). Cells were treated three days post-harvest with a fluorescent cobalamin prepared as described in Example 1. Differential uptake of fluorescent cobalamin analogues, as determined by fluorescence microscopy or fluorescence flow cytometry, in normal and leukemic human bone marrow cells was found. The difference between normal marrow cells and leukemic myeloblasts (cancer cells) is particularly noteworthy, with no detectable cobalamin being taken up by normal cells. A bone marrow sample from a healthy individual showed no fluorescent labeling. Uptake of a doxombicin-cobalamin conjugate, originally synthesized as a potential chemotherapeutic compound, was seen in P-388 murine leukemia cells and in HCT-116 human colon tumor cells. These results illustrate the uptake of fluorescent derivatives of cobalamin in leukemia and solid tumor cell lines.

### EXAMPLE 3

### Preparation of Cyanocobalamin Monocarboxylic Acids

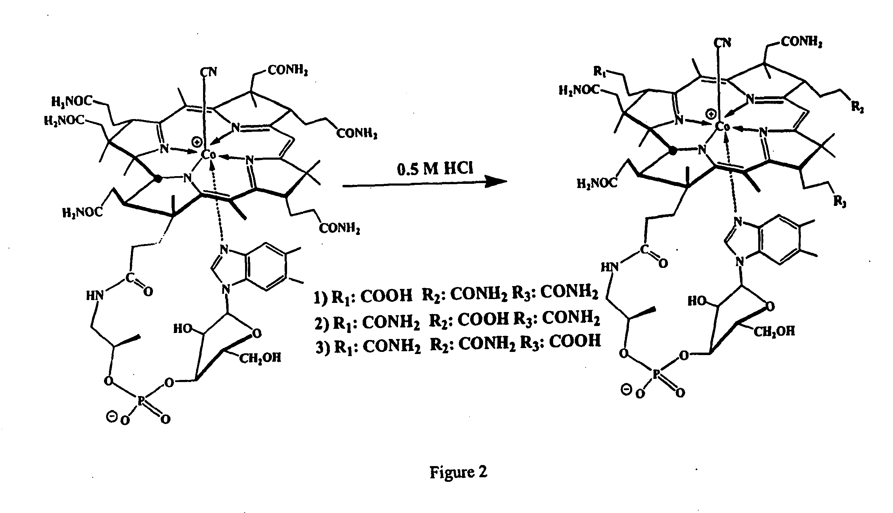

The b-, d-, and e-monocarboxylic acids were prepared by acid-catalyzed hydrolysis of cyanocobalamin. See Figure 2. Briefly, cyanocobalamin (527.0 mg, 0.389 mmol) was placed into a 100 ml round bottom flask and dissolved in 40 ml of 0.5 M HCl. The flask was placed in a water bath at 50 °C and stirred for 4 hours. The reaction was monitored via HPLC (Waters, Inc. 3.9 x 300mm DeltaPak 100 C-18 column) using the gradient tabulated in Table 1.

**TABLE 1**

| Time (min) | Flow Rate (ml/min) | 0.5 M H₃PO₄ (pH 3.0 w/NH₃OH) | 9:1 CH₃CN: H₂O |
|---|---|---|---|
| 0.0 | 2.0 | 90.0 | 10.0 |
| 2.0 | 2.0 | 90.0 | 10.0 |
| 18.0 | 2.0 | 83.7 | 16.3 |
| 23.0 | 2.0 | 30.0 | 70.0 |
| 25.0 | 2.0 | 30.0 | 70.0 |
| 30.0 | 2.0 | 90.0 | 10.0 |

After 4 hours the reaction was cooled to room temperature. The pH was adjusted to 7.0 with NaOH (10%) using a pH meter. The crude material was desalted using a C-18 SepPak column (Waters, Inc. P/N WATO23635) by first rinsing the column with 10 ml methanol followed by 15 ml deionized H₂O. The crude material was applied to the column via a syringe and rinsed with 10-15 ml deionized H₂O followed by elution with 10 ml methanol. The methanol was removed via rotary evaporation and a red compound was obtained (5016-12-33).

The crude reaction mixture was dissolved in minimal deionized H₂O and half of the solution was injected onto a semi-preparative HPLC (Waters, Inc. 25.0x300mm 100 C-18 column) using the gradient calculated in Table 2.

**TABLE 2**

| Time (min) | Flow Rate (ml/min) | 0.5 M H₃PO₄ (pH3.0w/ NH₃OH) | 9:1 CH₃CN: H₂O |
|---|---|---|---|
| 0.0 | 40.0 | 90.0 | 10.0 |
| 4.1 | 40.0 | 90.0 | 10.0 |
| 37.0 | 40.0 | 83.7 | 16.3 |
| 47.3 | 40.0 | 30.0 | 70.0 |
| 51.4 | 40.0 | 30.0 | 70,0 |
| 61.6 | 40.0 | 90.0 | 10.0 |

Peaks at 28.0 min (b-monocarboxylic acid, CBC-195), 30.1 min (d-monocarboxylic acid, CBC-226) and 34.6 min (e- monocarboxylic acid) were collected using large test tubes. The pure fractions were diluted 1:1 with deionized H₂O and desalted in the same method above. In all cases, a red solid was obtained.

CBC-195 (b-monocarboxylic acid): In the two preparative runs, 74.8 mg of the b-monocarboxylic acid (14.4 %) was isolated. A positive-ion electrospray mass spectrum (ES⁺) was obtained that shows a M+1 peak (1356) and a M+22 peak (1378) as expected. The b-monocarboxylic acid (CBC-195) was obtained in an overall yield of 14%

CBC-226 (d-monocarboxylic acid): In the two prep. runs, 38.6 mg of the d-monocarboxylic acid (7.3%) was isolated. A positive-ion electrospray mass spectrum (ES⁺) was obtained showing a M+1 peak (1356) and the corresponding M+Na peak (1378) as expected. The d-monocarboxylic acid (CBC-226) was obtained in an overall yield of 7%

The e-monocarboxylic acid was isolated, ~78 mg in an overall yield of 14%.

### EXAMPLE 4

### Conjugation of CNCbl Acids with 1. 12 Diaminododecane

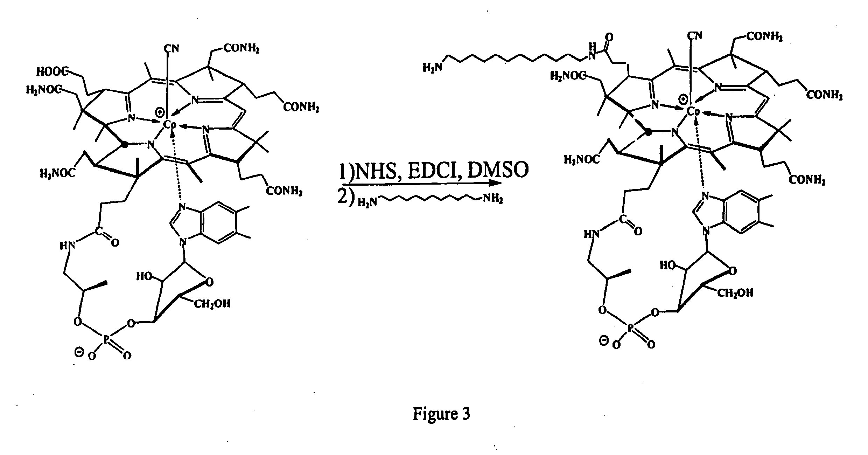

The b- and d- amines were prepared as shown in Figure 3. CBC-195 (55.4 mg, 0.0408 mmol) was added to a small glass vial and dissolved in ~2.5 ml of DMSO followed by the addition of EDCIHCI (12mg, 0.0626 mmol) and N-hydroxysuccinimide (NHS) (25 mg, 0.217 mmol). The reaction was stirred at room temperature overnight. From previous attempts, several equivalents of EDCI and NHS (a total of 6 equivalents) were required to drive the reaction to completion. After 24 hours, one additional equivalent of EDCI was added and the reaction was complete in a total of 26 hours. The reaction was monitored via HPLC using the gradient is Table 3. CBC-195 has a retention time of 9.07 min and the NHS-ester of CBC-195 has a retention time of 10.55 min.

**TABLE 3**

| Time (min) | Flow Rate (ml/min ) | 0.5 M H₃PO₄ (pH 3.0 w/ NH₃OH) | 9:1 CH₃CN: H₂O |
|---|---|---|---|
| 0.0 | 2.0 | 90.0 | 10.0 |
| 2.0 | 2.0 | 90.0 | 10.0 |
| 20.0 | 2.0 | 55.0 | 45.0 |
| 25.0 | 2.0 | 9.0 | 10.0 |

In a separate glass vial, 1,12-diaminododecane (81.8 mg, 0.408 mmol) was dissolved in ∼2 ml DMSO. The above reaction mixture was added dropwise using a syringe pump at 4.0 ml/hr to minimize dimerization. The product was formed immediately and has a retention time of 14.56 min. The crude reaction mixture was added to 100 ml of 1:1 CH₂Cl₂:Et₂O and a red precipitate formed. The red compound was filtered using a glass frit and washed with two 20 ml portions of CH₂Cl₂, two 20 ml portions of acetone, and finally by two 20 ml portions of Et₂O.

The crude reaction product was dissolved in a minimal amount of deionized H₂O and the solution was injected onto a semi-preparative HPLC (Waters, Inc., 25.0x100mm 100 C-18 column) using the gradient calculated in Table 4.

**TABLE 4**

| Time (min) | Flow Rate (ml/min ) | 0.5 M H₃PO₄ (pH 3.0 w/NH₃OH) | 9:1 CH₃CN; H₂O |
|---|---|---|---|
| 0.0 | 40.0 | 90.0 | 10.0 |
| 2.0 | 40.0 | 90.0 | 10.0 |
| 13.7 | 40.0 | 55.0 | 45.0 |
| 17.1 | 40.0 | 90.0 | 10.0 |

The peak at 8.70 min (b-amine, CBC-208) was collected using large test tubes. The pure fractions were diluted 1:1 with distilled H₂O and desalted using a C-18 SepPak column (Waters, Inc. P/N WAT023635) by first rinsing the column with 10 ml methanol followed by 15 ml deionized H₂O. The pure material was applied to the column via a syringe and rinsed with 10-15 ml deionized H₂O followed by elution with 10 ml methanol. The methanol was removed via rotary evaporation and 6 mg of a red compound was obtained.

CBC-208 (b-amine): A total of 6.0 mg of the b-amine was isolated. A positive-ion electrospray mass spectrum (ES⁺) was obtained that shows a M+1 peak (1538) and a M+23 peak (1560) as expected. CBC-208 was obtained in a yield of 9.5% after purification.

CBC-226 (d-amine): The d-monocarboxylic acid has an HPLC retention time of 9.32 mm, the NHS-ester migrates at 10.96 min, and the d-amine (CBC-226) migrates at 14.93 min using the same HPLC gradient as in Table 3. A positive-ion electrospray mass spectrum (ES⁺) was obtained of the crude material showing a M+1 peak (1538) and the corresponding M+Na peak (1560) as expected.

### EXAMPLE 5

### Ex vivo Examination of Breast Tumor Tissue via Microscopy

Samples of malignant and benign tumors, including tumors of the breast, with attached normal margin tissue are excised from patients. These samples are taken with approval of the University of Utah Institutional Review Board (IRB) and the Huntsman Cancer Institute Clinical Cancer Investigation Committee (CCIC). The live tissue samples are incubated with one of the fluorescent cobalamin derivatives prepared above for 4-6 hours. Thin tissue sections of each sample are prepared with a cryomicrotome and the amount of fluorescent marker is quantified in normal and cancerous tissue by epifluorescence microscopy. Corresponding tissue sections are stained with hematoxylin/eosin (H&E) stain for evaluation by an anatomical pathologist. The interface between normal and cancerous cells is examined carefully. Cells from the interior of the tumor are also examined for uptake of fluorescent marker, since cells within hypoxic regions of a tumor often have decreased metabolism.

More specifically, Minimum Essential Medium, alpha modification (α-MEM; 7.5% newborn calf serum, 2.5% fetal bovine serum, 0.2% nystatin, 2.5% penicillin/streptomycin, pH7.2; Sigma) was prepared and aliquoted (10 mL) into sterile 25 mL screw top tissue culture flasks. The media was brought to 37 °C, and tissue samples were incubated with fluorescently labeled cobalamins (50 nM; cobalamin-Oregon Green and cobalamin-naphthofluorescein conjugates of Example 1 and recombinant human TCII (50 pM) in α-MEM for 3 hours. Human breast tissue samples were procured under an IRM-approved protocol. The tissue was removed from the flask, washed with Dulbecco's Phosphate Buffered Saline (DPBS; Sigma), and mounted on a brass plate at -20 °C with OCT compound (Shandon) for frozen section slicing. Tissue was sliced (4-6 µm sections) in a CTD Harris cryostat at -20 °C. Thin tissue sections were pulled back with a small artist brush and fixed to a microscope slide with 100% ethanol. Slides were stained using a standard hematoxylin staining procedure: 95% ethanol, 20 seconds; water, 5 seconds; hematoxylin (Fisher), 45 seconds; water, 5 seconds; bluing solution (tap water), 10 seconds; 95% ethanol, 10 seconds; 100% ethanol, 10 seconds; xylene, 10 seconds; and xylene, 10 seconds. Slides were evaluated by phase contrast and epifluorescence microscopy at 10x, 60x and 100x magnification.

3-[4,5-Dimethylthiazol-2-yl]-2,5-diphenyltetrazolium thiazolyl bromide (MTT; Sigma) was used to qualitatively determine the metabolic competency of the tissue after 3 hours incubation time with fluorescent cobalamin. A portion of the tissue was removed from the media, washed with DPBS, and immersed in MTT (2 mL; 2.5 mg/mL). This tissue was incubated for 3 hours under a 5% CO2 atmosphere at 37 °C. During this incubation period, viable cells in the tissue sample reduced the MTT dye to purple formazan by succinate dehydrogenase activity (Celis and Celis, 1998). The tissue was washed with DPBS and prepared according to the cryomicrotome procedure outlined above to ensure the metabolic competency of the tissue.

The fluorescent cobalamin bioconjugates accumulated to some extent in both neoplastic and healthy breast tissue, with the neoplastic breast tissue sequestering more fluorescent cobalamin than healthy breast tissue. The amount of fluorescent cobalamin sequestered by healthy breast tissue is larger than expected, but it is believed that it is due to non-specific binding to structures within connective tissue rather than to significant internalization by healthy cells.

### EXAMPLE 6

### Ex vivo Examination of Cancer Cells in Lymph Nodes

Excised lymph nodes with metastatic disease are removed from patients and incubated for 4-8 hours with one of the fluorescent cobalamin derivatives prepared above. Each lymph node is sectioned and examined microscopically for transport of the fluorescent cobalamin into cancer cells. This experiment showed the ability of metastatic cells within lymph nodes to take up sufficient fluorescent cobalamin for imaging and visualization.

### EXAMPLE 7

### Use of Fluorescent Cobalamin to determine whether a patient will respond favorably to chemotherapy with a cobalamin-based therapeutic bioconjugate

A bone marrow aspirate or a peripheral blood sample from a patient with leukemia is incubated with a fluorescent cobalamin conjugate. After 4-8 hours, bone marrow aspirate or peripheral blood sample is washed to remove unincorporated fluorescent label and the cell sample subjected to qualitative or quantitative fluorescence analysis by epifluorescence microscopy or flow cytometry. Cells that have taken up a significant amount of fluorescent cobalamin exhibit a brighter fluorescence. The uptake of a significant amount of fluorescent cobalamin indicates that the type of leukemia the patient has will respond favorably to treatment with a cobalamin-based therapeutic. A bone marrow aspirate or a peripheral blood sample that does not show significant fluorescence after treatment with a fluorescent cobalamin conjugate indicates that the patient will not respond favorably to a cobalamin-based therapeutic conjugate. A similar approach can be applied to solid tumors. In this case, a portion of the excised tumor tissue is incubated with the fluorescent cobalamin conjugate and, after about 4-8 hours, fluorescence in the tumor tissue is quantified. The greater fluorescence exhibited by the tumor tissue, the greater the likelihood that the cancer will respond favorably to treatment with a cobalamin-based chemotherapeutic.

### LIST OF REFERENCES

Channel, R. (1975). "Extreme Elevation of Serum Transcobalamin I in Patients with Metastatic Cancer." New Engl. J. Med 292:282-284.
Celis, A. and Celis, J.E. (1998). Cell Biology, pp. 9-11.
Collins, D. A. and Hogenkamp, H. P. C. (1997). "Transcobalamin II Receptor Imaging via Radiolabeled Diethylene-Triaminepentaacetate Cobalamin Analogs." J. Nucl. Med. 38:717-723.
Collins, D.A. et aL (1999). "Tumor Imaging via Indium-111-Labeled DTPA-Adenosylcobalamin." Mayo Clinic Proceedings 74: 687-691.
Collins, D.A. et al. (2000). "Biodistribution of Radiolabeled Adenosylcobalamin in Patients Diagnosed with Various Malignancies." Mayo ClinicProceedings 75:568-580.
Flodh, H. (1968). "Accumulation of labelled Vitamin B-12 in Some Transplanted Tumors." Acta Ratiol. Suppl. 284:55-60.
Hogenkamp, H. P.C., et al. (1999). "The Pharmacological Uses of Cobalamin Bioconjugates." In The Chemistry and Biochemistry of B-12, Banerjee, R., Ed., John Wiley & Sons, New York, pp. 385-410.
Howard, W.A. et al. (1997). "Sonolysis Promotes Indirect C-Co Bond Cleavage of Alkylcob(III)alamins." Bioconj. Chem. 8:498-502.
McGreevy, J. M. (1998). "Sentinel Lymph Node Biopsy in Breast Cancer." Curr. Surg. 55:301-4.
Mitchell, A. M. et al. (1999). "targeting Leukemia Cells with Cobalamin Bioconjugates" In Enzymatic Mechanisms, Frey, P. A.; Northrop, D. B., Eds., pp 150-154.
McMasters, K. M. et al. (1999). "Sentinel Lymph Node Biopsy for Breast Cancer -- Not yet the Standard of Care." New England J. Med. 339:990.
Morton, D. L. et al. (1992). "Technical Details of Intraoperative Lymphatic Mapping for Early Stage Melanoma." Arch. Surg. 127:392-9.
Rachmilewitz, B, et al. (1971). , "Serum Transcobalamin in Myeloid Leukemia." J. Lab. Clin. Med. 78:275.
Schneider, Z. and Stroinski, A. (1987). Comprehensive B12, de Gruyter, Berlin, pp. 358.

## Claims

1. A cobalamin having the general formula (I) 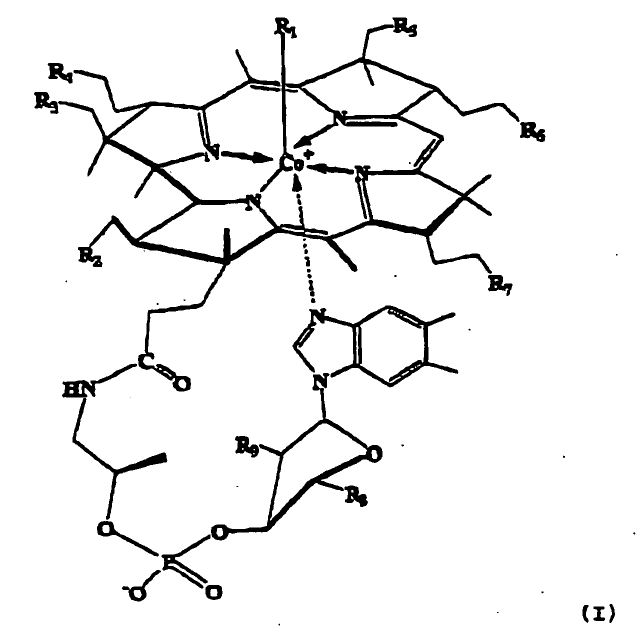 where R₁ is CN, OH, OH₂, CH₃, 5'-deoxyadeuosyl or (CH₂)ₚNHC(=S)Y; R₂, R₃, R₄, R₅, R₆ and R₇ are CONH₂; R₈ is CH₂OH or O(C=O)XₘY; R₉ is OH or O(C=O)XₘY; X is a linker having the formula NH(CH₂)ₙNHO(C=O); Y is a fluorophore, a phosphorophore, or a chemiluminescent chromophore; m is o or 1, n is 0-50 and p is 2-10, with the proviso that at least one of R₁, R₈ and R₉ groups has Y, wherein said cobalamin fluoresces, phosphoresces or luminesces when illuminated with ultraviolet, visible, or infrared light without cleavage of Y from the cobalamin.

2. The cobalamin according to claim 1, wherein the fluorophore, phosphorophore or chemiluminescent chromophore is attached to R₈ or R₉.

3. The cobalamine according to claim 1, wherein the fluorophore, phosphorophore or chemiluminescent chromophore is attached to R₁.

4. A cobalamin according to any of claims 1 to 3 for use as a diagnostic agent.

5. The use of a cobalamin according to any of claims 1-3 for preparing a diagnostic product for the identification of cancer tissue or tissue containing cancerous cells of an individual by contacting the cobalamin of any one of claims 1-3 with tissue suspected of being cancerous or containing cancerous cells, illuminating said tissue with light, and detecting the emitted fluorescence, phosphorescence or luminescence.

6. The use according to claim 5, wherein said tissue suspected of containing cancerous cells is a lymph node.

7. The use according to claim 6, wherein said lymph node is a sentinel lymph node or an axillary lymph node.

8. The use according to claim 5, wherein the identification is performed microscopically or visually.

9. The use according to claim 5, wherein said sample is obtained from said individual by biopsy.

10. The use of the cobalamin according to any of claims 1-3 for preparing a diagnostic product for visually differentiating cancerous tissue from healthy tissue of an individual by contacting the cobalamin of any one of claims 1-3 with tissue from said individual, illuminating said tissue and visually detecting fluorescence, phosphorescence or luminescence, whereby said cancerous tissues fluoresces, phosphoresces or luminesces and said healthy tissue exhibits less fluorescence, phosphorescence or luminescence.

11. A method for defining tumour margins *in vitro* or *ex vivo* which comprises contacting the cobalamin of any one of claims 1-3 with tissue from said individual suspected of containing a tumour, illuminating said tissue and detecting fluorescence, phosphorescence or luminescence, whereby said tumour tissue fluoresces, phosphoresces or luminesces and defines the margin of the tumour.

12. The use of a cobalamin according to any of claims 1-3 for preparing a diagnostic product for identifying metastatic cancer in an individual by contacting the cobalamin of any one of claims 1-3 with tissue or cells suspected of being metastatic cancer from said individual, illuminating said tissue and detecting florescence, phosphorescence or luminescence, whereby said metastatic cancer tissue or cells fluoresce, phosphoresce or luminesce.

13. The use according to claim 12, wherein the identification is performed visually or microscopically.

14. The use of a cobalamin according to any of claims 1-3 for preparing a product for diagnosing, detecting, predicting or monitoring cancer *in vivo, ex vivo* or *in situ* by contacting the fluorescent cobalamin of any one of claims 1-3 with tissue or cells from said individual, illuminating said tissue and detecting fluorescence, phosphorescence or luminescence, whereby cancer tissue or cells fluoresce, phosphoresce or luminesce and healthy tissue exhibits less fluorescence, phosphorescence or luminescence.

15. The use according to claim 14, wherein the contacting is performed in the course of a clinical pathology evaluation of tissue and cells.

16. The use of a cobalamin according to any of claims 1-3 for preparing a product for identifying metastatic disease in the treatment, diagnosis, detection, prediction, prognostication or monitoring of cancer in an individual by contacting the cobalamin of any one of claims 1-3 with tissue or cells from said individual, illuminating said tissue and detecting fluorescence, whereby cancer tissue or cells fluoresce, phosphoresce or luminesce and healthy tissue exhibits less fluorescence, phosphorescence or luminescence.

17. The use according to claim 16, wherein said cancer is breast cancer, colon cancer, ovarian cancer, lung cancer, prostate cancer, liver cancer, melanoma or a carcinoma that has spread via the lymphatic system.

18. The use according to claim 16, wherein said cancer is lymphoma or leukaemia.

19. The use according to claim 18, wherein said tissue is bone marrow aspirate or peripheral blood.

20. The use according to claim 16, which utilises flow cytometry or automatic analysis of body fluids.

21. The use of a cobalamin according to any of claims 1-3 for preparing a product for predicting the response of cancer cells to treatment by contacting the cobalamin of any one of claims 1-3 with cancer cells, illuminating said cells and detecting fluorescence, whereby cancer cells which exhibit a greater fluorescence, phosphorescence or luminescence than non-cancerous cells will respond favourably to treatment with a cobalamin-based chemotherapeutic agent.

22. The use according to claim 21, wherein said treatment is chemotherapy.

23. The use according to claim 22, wherein said chemotherapy utilises a cobalamin-therapeutic conjugate.

24. The use according to claim 21, wherein said treatment is hormonal therapy.

25. The use of a cobalamin according to any of claims 1-3 for preparing a product for determination of the stage of cancer progression by contacting the cobalamin of any of claims 1-3 with cancer cells, illuminating said cancer cells and detecting fluorescence, phosphorescence or luminescence, whereby the response of cancer cells to cobalamin-based therapy is directly proportional to the fluorescence, phosphorescence or luminescence of said cancer cells compared to non-cancerous cells.

26. A method to assay an amount of cobalamin in a sample in vitro which comprises performing a competitive binding assay on said sample using a cobalamin of any one of claims 1-3 and determining the amount of cobalamin present in said sample.

27. A method to assay an amount of unsaturated cobalamin binding capacity in a sample in vitro which comprises performing a competitive binding assay on cobalamin binding proteins isolated from said sample using a cobalamin of any one of claims 1-3 and determining the amount of unsaturated cobalamin binding capacity in said sample.

28. A method to assay an amount of cobalamin bound to cobalamin binding proteins in a sample in vitro which comprises performing a competitive binding assay of cobalamin separated from cobalamin binding proteins isolated from said sample using a cobalamin of any one of claims 1-3 and determining the amount of cobalamin bound to said proteins in said sample.

## Patentansprüche

1. Cobalamin der allgemeinen Formel (I) 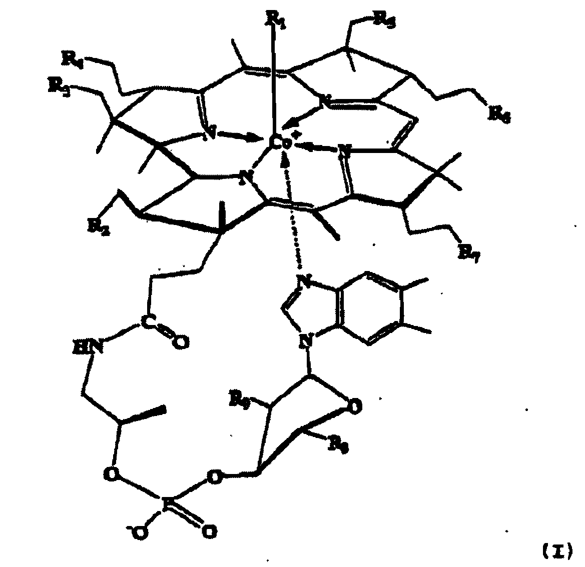 wobei R₁ CN, OH, OH₂, CH₃, 5'-Desoxyadenosyl oder (CH₂)pNHC(=S)Y ist; R₂, R₃, R₄, R₅, R₆ und R₇ CONH₂ sind; R₈ CH₂OH oder O(C=O)XₘY ist; R₉ OH oder O(C=O)XₘY ist; X ein Linker mit der Formel NH(CH₂)ₙNHO(C=O) ist; Y ein Fluorophor, ein Phosphorophor oder ein chemilumineszierender Chromophor ist; m 0 oder 1, n 0-50 und p 2-10 ist, mit der Maßgabe, dass von den Gruppen R₁, R₈ und R₉ mindestens eine Y aufweist, wobei das Cobalamin fluoresziert, phosphoresziert oder luminesziert, wenn es ohne Spaltung von Y von dem Cobalamin mit ultraviolettem, sichtbarem oder infrarotem Licht bestrahlt wird.

2. Cobalamin gemäß Anspruch 1, wobei der Fluorophor, Phosphorophor oder chemilumineszierende Chromophor an R₈ oder R₉ gebunden ist.

3. Cobalamin gemäß Anspruch 1, wobei der Fluorophor, Phosphorophor oder chemilumineszierende Chromophor an R₁ gebunden ist.

4. Cobalamin gemäß einem der Ansprüche 1 bis 3 zur Verwendung als diagnostisches Mittel.

5. Verwendung eines Cobalamins gemäß einem der Ansprüche 1 bis 3 zur Herstellung eines diagnostischen Produkts zum Identifizieren von Krebsgewebe oder kanzeröse Zellen enthaltendem Gewebe eines Individuums, indem das Cobalamin nach einem der Ansprüche 1 bis 3 mit Gewebe in Kontakt gebracht wird, von dem vermutet wird, dass es kanzerös ist oder kanzeröse Zellen enthält, das Gewebe mit Licht beleuchtet und die emittierte Fluoreszenz, Phosphoreszenz oder Lumineszenz detektiert wird.

6. Verwendung gemäß Anspruch 5, wobei das Gewebe, von dem vermutet wird, dass es kanzeröse Zellen enthält, ein Lymphknoten ist.

7. Verwendung gemäß Anspruch 6, wobei der Lymphknoten ein Wächterlymphknoten oder ein Achsellymphknoten ist.

8. Verwendung gemäß Anspruch 5, wobei die Identifizierung mikroskopisch oder visuell durchgeführt wird.

9. Verwendung gemäß Anspruch 5, wobei die Probe durch eine Biopsie vom Individuum erhalten wird.

10. Verwendung des Cobalamins gemäß einem der Ansprüche 1 bis 3 zur Herstellung eines diagnostischen Produkts zum optischen Unterscheiden zwischen Krebsgewebe und gesundem Gewebe bei einem Individuum, indem das Cobalamin nach einem der Ansprüche 1 bis 3 mit Gewebe des Individuums in Kontakt gebracht, das Gewebe beleuchtet und die Fluoreszenz, Phosphoreszenz oder Lumineszenz optisch detektiert wird, wobei das Krebsgewebe fluoresziert, phosphoresziert oder luminesziert und das gesunde Gewebe weniger Fluoreszenz, Phosphoreszenz oder Lumineszenz aufweist.

11. Verfahren zum Definieren von Tumorrändern *in vitro* oder *ex vivo*, welches das Kontaktieren des Cobalamins nach einem der Ansprüche 1 bis 3 mit Gewebe des Individuums, von dem vermutet wird, dass es einen Tumor enthält, das Beleuchten des Gewebes und das Detektieren der Fluoreszenz, Phosphoreszenz oder Lumineszenz umfasst, wobei das Tumorgewebe fluoresziert, phosphoresziert oder luminesziert und den Rand des Tumors definiert.

12. Verwendung eines Cobalamins gemäß einem der Ansprüche 1 bis 3 zur Herstellung eines diagnostischen Produkts zum Identifizieren von metastatischem Krebs bei einem Individuum, indem das Cobalamin nach einem der Ansprüche 1 bis 3 mit Gewebe oder Zellen des Individuums, von dem vermutet wird, dass es sich dabei um einen metastatischen Krebs handelt, in Kontakt gebracht, das Gewebe beleuchtet und die Fluoreszenz, Phosphoreszenz oder Lumineszenz detektiert wird, wobei das metastatische Krebsgewebe oder die Krebszellen fluoreszieren, phosphoreszieren oder lumineszieren.

13. Verwendung gemäß Anspruch 12, wobei die Identifizierung visuell oder mikroskopisch durchgeführt wird.

14. Verwendung eines Cobalamins gemäß einem der Ansprüche 1 bis 3 zur Herstellung eines Produkts zum Diagnostizieren, Detektieren, Prognostizieren oder Überwachen von Krebs *in vivo*, *ex vivo* oder *in situ*, indem das fluoreszierende Cobalamin nach einem der Ansprüche 1 bis 3 mit Gewebe oder Zellen des Individuums in Kontakt gebracht, das Gewebe beleuchtet und die Fluoreszenz, Phosphoreszenz oder Lumineszenz detektiert wird, wobei Krebsgewebe oder -zellen fluoreszieren, phosphoreszieren oder lumineszieren und gesundes Gewebe weniger Fluoreszenz, Phosphoreszenz oder Lumineszenz aufweist.

15. Verwendung gemäß Anspruch 14, wobei das Kontaktieren im Verlauf einer klinischen Pathologieauswertung von Gewebe und Zellen durchgeführt wird.

16. Verwendung eines Cobalamins gemäß einem der Ansprüche 1 bis 3 zur Herstellung eines Produkts zum Identifizieren einer metastatischen Erkrankung bei der Behandlung, Diagnose, Detektion, Vorhersage, Prognose oder Überwachung von Krebs bei einem Individuum, indem das Cobalamin nach einem der Ansprüche 1 bis 3 mit Gewebe oder Zellen des Individuums in Kontakt gebracht, das Gewebe beleuchtet und die Fluoreszenz detektiert wird, wobei Krebsgewebe oder -zellen fluoreszieren, phosphoreszieren oder lumineszieren und gesundes Gewebe weniger Fluoreszenz, Phosphoreszenz oder Lumineszenz aufweist.

17. Verwendung gemäß Anspruch 16, wobei es sich bei dem Krebs um Brustkrebs, Dickdarmkrebs, Eierstockkrebs, Lungenkrebs, Prostatakrebs, Leberkrebs, Melanom oder ein Karzinom handelt, das sich über das lymphatische System ausgebreitet hat.

18. Verwendung gemäß Anspruch 16, wobei es sich bei dem Krebs um ein Lymphom oder Leukämie handelt.

19. Verwendung gemäß Anspruch 18, wobei das Gewebe abgesaugtes Knochenmark oder peripheres Blut ist.

20. Verwendung gemäß Anspruch 16, wobei eine Durchflusszytometrie oder automatische Analyse von Körperflüssigkeiten eingesetzt wird.

21. Verwendung eines Cobalamins gemäß einem der Ansprüche 1 bis 3 zur Herstellung eines Produkts zum Prognostizieren der Reaktion von Krebszellen auf eine Behandlung, indem das Cobalamin nach einem der Ansprüche 1 bis 3 mit Krebszellen in Kontakt gebracht wird, die Zellen beleuchtet werden und die Fluoreszenz detektiert wird, wobei Krebszellen, die eine stärkere Fluoreszenz, Phosphoreszenz oder Lumineszenz als nicht kanzeröse Zellen aufweisen, auf die Behandlung mit einem chemotherapeutischen Mittel auf Cobalaminbasis günstig ansprechen werden.

22. Verwendung gemäß Anspruch 21, wobei die Behandlung eine Chemotherapie ist.

23. Verwendung gemäß Anspruch 22, wobei bei der Chemotherapie ein Cobalamintherapeutisches Konjugat eingesetzt wird.

24. Verwendung gemäß Anspruch 21, wobei die Behandlung eine Hormontherapie ist.

25. Verwendung eines Cobalamins gemäß einem der Ansprüche 1 bis 3 zur Herstellung eines Produkts zur Bestimmung des Stadiums der Krebsentwicklung, indem das Cobalamin nach einem der Ansprüche 1 bis 3 mit Krebszellen in Kontakt gebracht wird, die Krebszellen beleuchtet werden und die Fluoreszenz, Phosphoreszenz oder Lumineszenz detektiert wird, wobei die Reaktion der Krebszellen auf eine Therapie auf Cobalaminbasis zur Fluoreszenz, Phosphoreszenz oder Lumineszenz der Krebszellen verglichen mit nicht kanzerösen Zellen direkt proportional ist.

26. Verfahren zum in-vitro-Analysieren einer Cobalaminmenge in einer Probe, welches das Durchführen eines kompetitiven Bindungsassays an der Probe unter Verwendung eines Cobalamins nach einem der Ansprüche 1 bis 3 und das Bestimmen der in der Probe vorhandenen Cobalaminmenge umfasst.

27. Verfahren zum in-vitro-Analysieren des Umfangs der ungesättigten Cobalamin-Bindefähigkeit in einer Probe, welches das Durchführen eines kompetitiven Bindungsassays an aus der Probe isolierten Cobalamin-Bindungsproteinen unter Verwendung eines Cobalamins nach einem der Ansprüche 1 bis 3 und das Bestimmen des Umfangs der ungesättigten Cobalamin-Bindefähigkeit in der Probe umfasst.

28. Verfahren zum in-vitro-Analysieren einer Menge an Cobalamin, das an Cobalamin-Bindungsproteine gebunden ist, in einer Probe, welches das Durchführen eines kompetitiven Bindungsassays an Cobalamin, das von aus der Probe isolierten Cobalamin-Bindungsproteinen abgetrennt wurde, unter Verwendung eines Cobalamins nach einem der Ansprüche 1 bis 3 und das Bestimmen der Menge an an diese Proteine gebundenem Cobalamin in der Probe umfasst.

## Revendications

1. Cobalamine ayant la formule générale (I) 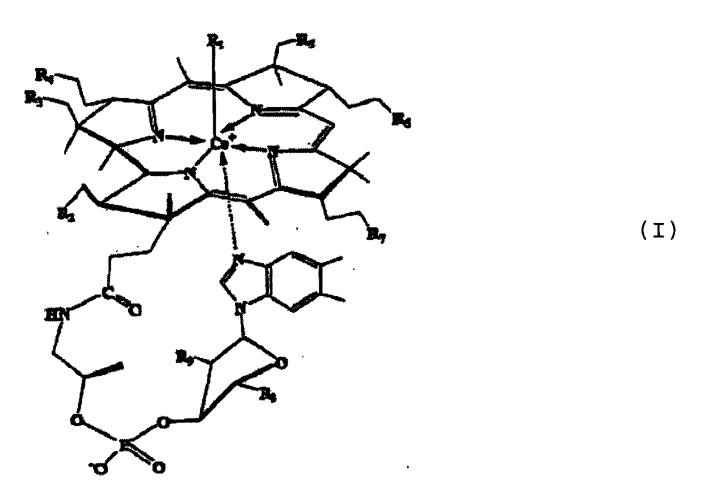 dans laquelle R₁ représente CN, OH, OH₂, CH₃, le 5'-désoxyadénosyle ou (CH₂)ₚNHC(=S))Y ; R₂, R₃, R₄, R₅, R₆ et R₇ représentent CONH₂ ; R₈ représente CH₂OH ou O(C=O)XₘY ; R₉ représente OH ou O(C=O)XₘY ; X représente un espaceur ayant la formule NH(CH₂)ₙNHO(C=O) ; Y représente un agent fluorescent, un agent phosphorescent, ou un chromophore chimiluminescent ; m représente 0 ou 1, n représente un nombre de 0 à 50 et p représente un nombre de 2 à 10, à condition qu'au moins un groupe parmi les groupes R₁, R₈ et R₉ possède un Y, ladite cobalamine étant fluo-rescente, phosphorescente ou luminescente lors-qu'elle est illuminée avec une lumière ultraviolette, visible, ou infrarouge sans coupure du Y de la cobalamine.

2. Cobalamine selon la revendication 1, dans laquelle l'agent fluorescent, l'agent phosphores-cent ou le chromophore chimiluminescent est lié à R₈ ou R₉.

3. Cobalamine selon la revendication 1, dans laquelle l'agent fluorescent, l'agent phosphores-cent ou le chromophore chimiluminescent est lié à R₁.

4. Cobalamine selon l'une quelconque des revendications 1 à 3 pour son utilisation en tant qu'agent de diagnostic.

5. Utilisation d'une cobalamine selon l'une quelconque des revendications 1 à 3 pour préparer un produit de diagnostic pour l'identification d'un tissu cancéreux ou d'un tissu contenant des cellules cancéreuses d'un individu en mettant en contact la cobalamine selon l'une quelconque des revendications 1 à 3 avec un tissus suspecté d'être cancéreux ou de contenir des cellules cancéreuses, en illuminant lesdit tissu avec de la lumière, et en détectant la fluorescence, la phosphorescence ou la luminescence émise.

6. Utilisation selon la revendication 5, dans laquelle ledit tissu suspecté de contenir des cellules cancéreuses est un ganglion lymphatique.

7. Utilisation selon la revendication 6, dans laquelle ledit ganglion lymphatique est un gan-glion lymphatique sentinelle ou un ganglion lymphatique axillaire.

8. Utilisation selon la revendication 5, dans laquelle l'identification est effectuée au microscope ou visuellement.

9. Utilisation selon la revendication 5, dans laquelle ledit échantillon est obtenu à partir dudit individu par biopsie.

10. Utilisation de la cobalamine selon l'une quelconque des revendications 1 à 3 pour préparer un produit de diagnostic pour différencier visuellement de tissu cancéreux d'un tissu sain d'un individu en mettant en contact la cobalamine selon l'une quelconque des revendications 1 à 3 avec un tissu provenant dudit individu, en illu-minant ledit tissu et en détectant visuellement la fluorescence, la phosphorescence ou la lumi-nescence, ledit tissu cancéreux étant fluorescent, phosphorescent ou luminescent et ledit tissu sain présentant moins de fluorescence, de phospho-rescence ou de luminescence.

11. Méthode pour définir les bords d'une tumeur *in vitro* ou *ex vivo* qui comprend la mise en contact de la cobalamine selon l'une quelconque des revendications 1 à 3 avec un tissu provenant dudit individu suspecté de contenir une tumeur, l'illumination dudit tissu et la détection de la fluorescence, de phosphorescence ou de lumines-cence, ledit tissu tumoral étant fluorescent, phosphorescent ou luminescent et définissant les bords de la tumeur.

12. Utilisation d'une cobalamine selon l'une quelconque des revendications 1 à 3 pour préparer un produit de diagnostic pour identifier un cancer métastatique chez un individu en mettant en contact la cobalamine selon l'une quelconque des revendications 1 à 3 avec un tissu ou des cellules suspectés d'être un cancer métastatique provenant dudit individu, en illuminant ledit tissu et en détectant la fluorescence, la phosphorescence ou la luminescence, ledit tissu ou lesdites cellules du cancer métastatique étant fluorescents, phosphorescents ou luminescents.

13. Utilisation selon la revendication 12, dans laquelle l'identification est effectuée visuelle-ment ou au microscope.

14. Utilisation d'une cobalamine selon l'une quelconque des revendications 1 à 3 pour préparer un produit pour diagnostiquer, détecter, prédire ou contrôler un cancer *in vivo*, *ex vivo* ou *in situ* en mettant en contact la cobalamine fluo-rescente selon l'une quelconque des revendica-tions 1 à 3 avec un tissu ou des cellules proven-ant dudit individu, en illuminant ledit tissu et en détectant la fluorescence, la phosphorescence ou la luminescence, ledit tissu ou lesdites cellules cancéreux étant fluorescents, phospho-rescents ou luminescents et ledit tissu sain présentant moins de fluorescence, de phosphores-cence ou de luminescence.

15. Utilisation selon la revendication 14, dans laquelle la mise en contact est effectuée au cours d'une évaluation d'anatomopathologie cli-nique d'un tissu et de cellules.

16. Utilisation d'une cobalamine selon l'une quelconque des revendications 1 à 3 pour préparer un produit pour identifier une maladie méta-statique dans le traitement, le diagnostic, la détection, la prédiction, le pronostic ou le suivi d'un cancer chez un individu en mettant en contact la cobalamine selon l'une quelconque des revendications 1 à 3 avec un tissu ou des cellules provenant dudit individu, en illuminant ledit tissu et en détectant la fluorescence, ledit tissu ou lesdites cellules cancéreux étant fluorescents, phosphorescents ou luminescents et ledit tissu sain présentant moins de fluorescence, de phosphorescence ou de luminescence.

17. Utilisation selon la revendication 16, dans laquelle ledit cancer est un cancer du sein, un cancer du colon, un cancer de l'ovaire, un cancer du poumon, un cancer de la prostate, un cancer du foie, un mélanome ou un carcinome qui s'est pro-pagé par le biais du système lymphatique.

18. Utilisation selon la revendication 16, dans laquelle ledit cancer est un lymphome ou une leucémie.

19. Utilisation selon la revendication 18, dans laquelle ledit tissu est un aspirat de moelle osseuse ou du sang périphérique.

20. Utilisation selon la revendication 16, qui utilise la cytométrie en flux ou une analyse automatique de fluides corporels.

21. Utilisation d'une cobalamine selon l'une quelconque des revendications 1 à 3 pour préparer un produit pour prédire la réponse de cellules cancéreuses à un traitement en mettant en contact la cobalamine selon l'une quelconque des revendi-cations 1 à 3 avec les cellules cancéreuses, en illuminant lesdites cellules et en détectant la fluorescence, moyennant quoi les cellules cancéreuses qui présentent une fluorescence, une phosphorescence ou une luminescence plus grande que les cellules non cancéreuses répondront favo-rablement à un traitement avec un agent chimio-thérapique à base de cobalamine.

22. Utilisation selon la revendication 21, dans laquelle ledit traitement est une chimiothérapie.

23. Utilisation selon la revendication 22, dans laquelle ladite chimiothérapie utilise un conju-gué thérapeutique de la cobalamine.

24. Utilisation selon la revendication 21, dans laquelle ledit traitement est une thérapie hormonale.

25. Utilisation d'une cobalamine selon l'une quelconque des revendications 1 à 3 pour préparer un produit pour déterminer le stade de la progression du cancer en mettant en contact la cobalamine selon l'une quelconque des revendica-tions 1 à 3 avec les cellules cancéreuses, en illuminant lesdites cellules cancéreuses et en détectant la fluorescence, la phosphorescence ou la luminescence, la réponse des cellules cancé-reuses à la thérapie à base de cobalamine étant directement proportionnelle à la fluorescence, phosphorescence ou luminescence desdites cellules cancéreuses par comparaison à des cellules non cancéreuses.

26. Méthode de dosage d'une quantité de cobal-amine dans un échantillon *in vitro* qui comprend la réalisation d'un dosage par liaison compéti-tive sur ledit échantillon utilisant une cobal-amine selon l'une quelconque des revendications 1 à 3 et la détermination de la quantité de cobal-amine présente dans ledit échantillon.

27. Méthode de dosage d'une quantité correspon-dant à la capacité de liaison de la cobalamine insaturée dans un échantillon *in vitro* qui comprend la réalisation d'un dosage par liaison compétitive sur des protéines se liant à la cobalamine isolées à partir dudit échantillon en utilisant une cobalamine selon l'une quelconque des revendications 1 à 3 et la détermination de la quantité correspondant à la capacité de liai-son de la cobalamine insaturée dans ledit échantillon.

28. Méthode de dosage d'une quantité de cobal-amine liée aux protéines se liant à la cobalamine dans un échantillon *in vitro* qui comprend la réalisation d'un dosage par liaison compétitive de la cobalamine séparée des protéines se liant à la cobalamine isolées à partir dudit échantillon en utilisant une cobalamine selon l'une quel-conque des revendications 1 à 3 et la détermination de la quantité de cobalamine liée auxdites protéines dans ledit échantillon.
